# EUROPEAN PATENT SPECIFICATION

(11) **EP 3 873 236 B1**
(45) Date of publication and mention of the grant of the patent: **26.02.2025**
(21) Application number: 19801243.7
(22) Date of filing: 01.11.2019
(51) Int. Cl.: A23L 33/16, A23L 33/175, A61K 31/198, A61K 45/06

(54) **POWDERS CONTAINING A BUFFER SALT AND AN AMINO ACID, RECONSTITUTION OF SUCH A POWDER INTO A NUTRITIONAL PRODUCT, AND METHODS OF USING SUCH A NUTRITIONAL PRODUCT**
PULVER, DAS EIN PUFFERSALZ UND EINE AMINOSÄURE ENTHÄLT, REKONSTITUTION EINES SOLCHEN PULVERS ZU EINEM ERNÄHRUNGSPRODUKT UND VERFAHREN ZUR VERWENDUNG EINES SOLCHEN ERNÄHRUNGSPRODUKTES
POUDRE CONTENANT UN SEL TAMPON ET UN ACIDE AMINÉ, RECONSTITUTION D'UNE TELLE POUDRE EN UN PRODUIT NUTRITIONNEL, ET MÉTHODES D'UTILISATION D'UN TEL PRODUIT NUTRITIONNEL

(30) Priority: 02.11.2018 US 201862754918 P
(43) Date of publication of application: 08.09.2021
(73) Proprietor: Société des Produits Nestlé S.A., 1800 Vevey (CH)
(72) Inventor: GEMILI, Seyhun, Bedminster, NJ 07921 (US)
(74) Representative: Gordon, Kirsteen Helen
(86) International application number: PCT/EP2019/079947
(87) International publication number: WO 2020/089447

(56) References cited:
- EP-A1- 2 374 452
- WO-A1-2004/107881
- WO-A1-2012/013975
- WO-A1-2016/033183
- WO-A1-2016/191468
- US-A- 5 629 023
- US-A1- 2006 269 535
- US-A1- 2009 176 715
- US-A1- 2011 077 303
- US-A1- 2015 132 440

## Description

### BACKGROUND

The present disclosure generally relates to compositions that use buffer salt, amino acid, and at least certain vitamins and minerals. The composition is a powder comprising a phosphate salt, at least one glycine or functional derivative thereof, at least one N-acetylcysteine, L-cysteine or functional derivative thereof, Vitamin E, Vitamin C, Vitamin B2 and Zinc, the powder comprising less than 1.0 wt.% of proteins of four or more amino acid residues, and the powder having at least one characteristic selected from the group consisting of (i) the amino acids in the powder are present in an amount that at least partially comprises the amino acid in a form selected from the group consisting of free form, dipeptides, tripeptides and mixtures thereof and (ii) the powder is substantially free of enzymes and antibodies. The powder can be reconstituted in a liquid to form a nutritional product.

Population aging has been a remarkable demographic event. As the growth of the older population has outpaced the total population due to increased longevity, the proportion of older persons relative to the rest of the population has increased considerably. For example, one in every twelve individuals was at least 60 years of age in 1950, and one in every ten was aged 60 years or older by the end of 2000. By the end of 2050, the number of persons worldwide that is 60 years or over is projected to be one in every five.

Aged or aging individuals frequently suffer some degree of cognitive impairment, including decline in cognitive function, that progresses with age, and age-related changes in brain morphology and cerebrovascular function are commonly observed. Cognitive decline has been consistently reported with aging across a range of cognitive domains including processing speed, attention, episodic memory, spatial ability and executive function. Brain imaging studies have revealed that these normal age-related cognitive declines are associated with decreases in both grey and white matter volume in the brain, with the fronto-striatal system most heavily compromised with aging. These decreases in cortical volume can be attributed to a number of detrimental cellular processes involved with normal aging, such as accumulation of damage by free radicals over time leading to oxidative damage, chronic low-grade inflammation, homocysteine accumulation (which when elevated are a risk factor for cognitive impairment and dementia), and decreased mitochondrial efficiency. In addition to direct cellular damage, the brain is also indirectly impaired by insults to micro-vascular structures.

It is evident that the pathology of aging and also dementia involves a complexity of these interacting factors which are linked together. For example, mitochondrial dysfunction leads to increased oxidative stress, and oxidative stress can trigger inflammation and vascular insults.

Furthermore, cognitive decline is an early predictor or Alzheimer pathology and begins before the onset of dementia. In this context, the cognitive composite score represents a reliable means to assess the cognitive decline preceding dementia. Considerable evidence suggests that maintaining brain health and preventing cognitive decline with advancing age may prevent or delay development of dementia due to Alzheimer's disease and other aged related neuropathologies.

In biology and psychology, the term "stress" refers to the consequence of the failure of a human or other animal to respond appropriately to physiological, emotional, or physical threats, whether actual or imagined. The psychobiological features of stress may present as manifestations of oxidative stress, i.e., an imbalance between the production and manifestation of reactive oxygen species and the ability of a biological system readily to detoxify the reactive intermediates or to repair the resulting damage. Disturbances in the normal redox state of tissues can cause toxic effects through the production of peroxides and free radicals that damage all of the components of the cell, including proteins, lipids, and DNA. Some reactive oxidative species can even act as messengers through a phenomenon called "redox signaling."

In humans, oxidative stress is involved in many diseases. Examples include atherosclerosis, Parkinson's disease, heart failure, myocardial infarction, Alzheimer's disease, schizophrenia, bipolar disorder, fragile X syndrome, and chronic fatigue syndrome.

One source of reactive oxygen under normal conditions in humans is the leakage of activated oxygen from mitochondria during oxidative phosphorylation. Other enzymes capable of producing superoxide (O2-) are xanthine oxidase, NADPH oxidases and cytochromes P450. Hydrogen peroxide, another strong oxidizing agent, is produced by a wide variety of enzymes including several oxidases. Reactive oxygen species play important roles in cell signaling, a process termed redox signaling. Thus, to maintain proper cellular homeostasis a balance must be struck between reactive oxygen production and consumption.

Oxidative stress contributes to tissue injury following irradiation and hyperoxia. It is also suspected to be important in neurodegenerative diseases, including Alzheimer's disease, Parkinson's disease, amyotrophic lateral sclerosis (ALS), and Huntington's disease.

Oxidative stress is also thought to be linked to certain cardiovascular diseases, since oxidation of low-density lipoprotein (LDL) in the vascular endothelium is a precursor to plaque formation. Oxidative stress also plays a role in the ischemic cascade due to oxygen reperfusion injury following hypoxia. This cascade includes both strokes and heart attacks. Oxidative stress has also been implicated in chronic fatigue syndrome.

Moreover, the free radical theory of aging suggests that the biological process of aging results in increased oxidative stress in elderly humans. The ability of a cell to resist the damaging potential of oxidative stress is determined by a vital balance between generation of oxidant free radicals and the defensive array of antioxidants available to the cell. There are multiple antioxidant defense systems and of these, glutathione (GSH) is the most abundant intracellular component of overall antioxidant defenses. GSH, a tripeptide, is synthesized from precursor amino-acids glutamate, cysteine, and glycine in two steps catalyzed by glutamate cysteine ligase (GCL, also known as gamma-glutamylcysteine synthetase, EC 6.3.2.2) and gamma-L-glutamyl-L-cysteine:glycine ligase (also known as glutathione synthetase, EC 6.3.2.3), and GSH synthesis occurs *de novo* in cells.

WO 2004/107881 A1 relates to a nutrient composition or combination of compositions for the treatment or prophylaxis of infections, in particular HIV/AIDS, and for the enhancement of immunity, based on selenium in synergistic combinations with biologically absorbable sources of glutathione, alkalinity enhancing components, a source of sulphur, an anti-mutagenic compound and for oral use, gastro-intestinal absorption enhancers.

US 5 629 023 A relates to a composition of an enteral nutrition product, which when taken alone, or in combination with certain conventional food sources, helps to upregulate hepatic detoxification enzyme systems thereby reducing the symptoms associated with endotoxicity and exotoxicity.

### SUMMARY

Acidic amino acids such as N-acetyl cysteine (NAC) can denature proteins in liquid. Existing solutions use basic additives such as potassium hydroxide and sodium hydroxide to increase the pH of protein-containing liquids. However, the present inventors noted that the increase in the pH of the protein-containing solution can change the configuration of the protein structures, which can lead to denaturation or gelation before the acidic amino acids are added.

The present inventors surprisingly found that buffering salts such as disodium phosphate and/or potassium phosphate dibasic can be blended with acidic amino acids and then added into liquids containing protein such as milk protein, plant protein, collagen or blends thereof. Thus denaturation of the proteins in different concentrations and conditions can be avoided by establishing and/or maintaining a stable pH of the liquid.

Indeed, proteins are sensitive to pH changes in the liquid environment, and even slight changes in the pH may cause disruption of protein structure and cause denaturation that could lead to gelation, precipitation and aggregation. Addition of acidic or basic components is one way that causes change in the pH of the environment. For example, amino acids have a chemical structure that could create the increase or decrease in the pH. Therefore, addition of these amino acids into protein containing liquid products could lead to denaturation due to increase or decrease of the pH. As set forth later herein, the present inventors evaluated addition of buffering salts to avoid drastic changes in the pH of protein containing liquid. The present inventors found that these buffering salts minimized the change in the pH and decreased the protein precipitation compared to cases at which no buffering salts were used.

Accordingly, the present disclosure provides a powder comprising a phosphate salt, at least one glycine or functional derivative thereof, at least one N-acetylcysteine, L-cysteine or functional derivative thereof, Vitamin E, Vitamin C, Vitamin B2 and Zinc, the powder comprising less than 1.0 wt.% of proteins of four or more amino acid residues, and the powder having at least one characteristic selected from the group consisting of (i) the amino acids in the powder are present in an amount that at least partially comprises the amino acid in a form selected from the group consisting of free form, dipeptides, tripeptides and mixtures thereof and (ii) the powder is substantially free of enzymes and antibodies..

In an embodiment, the powder further comprises at least one of calcium, sodium or potassium.

In an embodiment, the amino acids comprise L-cysteine, glycine, and L-glutamate that are provided by glutathione in the powder.

In an embodiment, the amino acid comprises a combination of at least one N-acetyl-cysteine or functional derivative thereof and at least one glycine or functional derivative thereof.

A method of decreasing or preventing protein denaturation and/or protein coagulation caused by reconstitution of a powder in a liquid comprising an ingredient selected from the group consisting of a protein, an acidic component, and a mixture thereof can be provided (not part of the invention). Such a method comprises a step selected from the group consisting of (i) dry mixing a buffer salt with an amino acid and (ii) drying an aqueous solution comprising a buffer salt and an amino acid. The powder is as hereinbefore described.

The amino acids comprise a combination of at least one N-acetyl-cysteine, L-cysteine or functional derivative thereof and at least one glycine or functional derivative thereof.

A liquid nutritional product (not part of the invention) can comprise a buffer salt, an amino acid, and an ingredient selected from the group consisting of an acidic component, a protein and mixtures thereof. Such a nutritional product can be made by a process comprising reconstituting a powder comprising the buffer salt and the amino acid into a liquid comprising the ingredient. The powder is as hereinbefore described.

The nutritional product can be a beverage.

The liquid can comprise an acidic component that is at least one of juice or a polyphenol (e.g., a flavonoid).

The liquid can comprise a protein selected from the group consisting of milk protein, plant protein, collagen and blends thereof.

A method of making a powder formulated for reconstitution into a liquid to form a nutritional product can be provided (not part of the invention). The liquid comprises an ingredient selected from the group consisting of a protein, an acidic component, and a mixture thereof. Such a method comprises a step selected from the group consisting of (i) dry mixing a buffer salt with an amino acid and (ii) drying an aqueous solution comprising a buffer salt and an amino acid. The powder is as hereinbefore described.

An advantage of one or more embodiments provided by the present disclosure is to facilitate reconstitution of an amino acid, such as N-acetyl cysteine, L-cysteine and/or glycine, by minimizing or preventing protein denaturation and coagulation in a liquid in which the amino acid is reconstituted, such as a liquid containing an acidic component that is at least one of juice or a polyphenol (e.g., a flavonoid) and/or containing protein such as milk protein, plant protein, collagen or blends thereof.

Another advantage of one or more embodiments provided by the present disclosure is to facilitate reconstitution of a powder containing a vitamin and/or a mineral that can increase precipitation, such as sodium, potassium or calcium, by minimizing or preventing the negative impact of such compounds on the dissolution of the powder in a liquid.

Additional features and advantages are described herein and will be apparent from the following Figures and Detailed Description.

### BRIEF DESCRIPTION OF DRAWINGS

**FIG. 1** shows the experimentally tested dissolution of powdered N-acetyl-cysteine (NAC) without buffer salts in a dairy-based liquid at room temperature as disclosed herein.
**FIG. 2** shows the experimentally tested dissolution of powdered NAC with buffer salts in a dairy-based liquid at room temperature as disclosed herein.
**FIG. 3** shows the experimentally tested dissolution of powdered NAC and glycine without buffer salts in a dairy-based liquid at room temperature as disclosed herein.
**FIGS. 4** shows the experimentally tested dissolution of powdered NAC and glycine with buffer salts in a dairy-based liquid at room temperature as disclosed herein.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified. A composition "consisting essentially of" contains at least 50 wt.% of the referenced components, preferably at least 75 wt.% of the referenced components, more preferably at least 85 wt.% of the referenced components, most preferably at least 95 wt.% of the referenced components.

The term "and/or" used in the context of "X and/or Y" should be interpreted as "X," or "Y," or "X and Y." Similarly, "at least one of X or Y" should be interpreted as "X," or "Y," or "X and Y." For example, "at least one glycine or functional derivative thereof" should be interpreted as "glycine," or "a functional derivative of glycine," or "both glycine and a functional derivative of glycine."

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

The term "nutritional product" as used herein means a composition that is intended for ingestion by an individual such as a human and provides at least one nutrient to the individual. The nutritional products of the present disclosure, including the many embodiments described herein, can comprise, consist of, or consist essentially of the elements disclosed herein, as well as any additional or optional ingredients, components, or elements described herein or otherwise useful in a diet.

As used herein, the term "isolated" means removed from one or more other compounds or components with which the compound may otherwise be found, for example as found in nature. For example, "isolated" preferably means that the identified compound is separated from at least a portion of the cellular material with which it is typically found in nature. In an embodiment, an isolated compound is pure, i.e., free from any other compound.

As used herein, "amino acid" includes the standard (proteinogenic) amino acids and also derivatives of the standard amino acids, such as compounds resulting from reaction at an amino group, carboxy group, or side-chain functional group of the amino acid or from the replacement of any hydrogen by a heteroatom. Non-limiting examples of suitable amino acid derivatives according to the present disclosure include amino acid esters such as methyl esters, amino acid tert-butyl esters, benzyl esters, and ethyl esters. An "acidic" amino acid is any amino acid or derivative thereof that is capable of donating a hydron (proton or hydrogen ion H+) or forming a covalent bond with an electron pair.

As used herein, a "powder" is a composition in which any moisture is no greater than 6 wt.% of the composition.

### Embodiments

An aspect of the present disclosure is a powder comprising a phosphate salt, at least one glycine or functional derivative thereof, at least one N-acetylcysteine, L-cysteine or functional derivative thereof, Vitamin E, Vitamin C, Vitamin B2 and Zinc, the powder comprising less than 1.0 wt.% of proteins of four or more amino acid residues, and the powder having at least one characteristic selected from the group consisting of (i) the amino acids in the powder are present in an amount that at least partially comprises the amino acid in a form selected from the group consisting of free form, dipeptides, tripeptides and mixtures thereof and (ii) the powder is substantially free of enzymes and antibodies. The powder can be formulated for reconstitution in a liquid to form a nutritional product. In an embodiment, the amino acid comprises L-cysteine, L-glutamic acid, and glycine that are provided by glutathione in the powder.

Subject to the requirement that the powder comprises Vitamin E, Vitamin C, and Vitamin B2, non-limiting examples of suitable vitamins which the powder can comprise include vitamin C and group B vitamins, and other non-limiting examples of suitable vitamins include ascorbic acid, ascorbyl palmitate, vitamins B1, B2, B6, B12, and Niacin (B3), or combination of thereof. The vitamins may also include Vitamins A, D, E and K and acid vitamins such as pantothenic acid, folic acid and biotin. The Vitamin A may be present as Vitamin A Palmitate and/or beta-carotene. Vitamin D3 is an example of a suitable form of Vitamin D, and Vitamin D2 can also be used in some embodiments.

Subject to the requirement that the powder comprises zinc, non-limiting examples of suitable minerals which the powder can comprise include calcium, sodium, magnesium, iron, zinc, iodine, copper, phosphorus, manganese, potassium, chromium, molybdenum, selenium, nickel, tin, silicon, vanadium and boron.

The powder can be administered in an amount that provides the recommended daily requirement (RDA) of the vitamin and/or mineral therein. In some embodiments, a unit dosage form of the powder contains a predetermined portion of the RDA for the vitamin and/or mineral, for example about 50% of the RDA, about 75% of the RDA, about 100% of the RDA, or values therebetween. For example, a unit dosage form of the powder can contain about 50% of the RDA for the vitamin and/or mineral such that administration of two of the unit dosage forms in one day provides approximately the entirety of the RDA for the corresponding vitamin and/or mineral. As another example, a unit dosage form of the powder can contain 100% of the RDA for the vitamin and/or mineral such that administration of one of the unit dosage forms provides approximately the entirety of the RDA for the corresponding vitamin and/or mineral.

The powder comprises Vitamin E, Vitamin C, Vitamin B2 and Zinc. Therefore, a preferred embodiment of the powder comprises a buffer salt, at least one glycine or functional derivative thereof, at least one N-acetylcysteine or functional derivative thereof, Vitamin E, Vitamin C, Vitamin B2 and Zinc, most preferably with the vitamins and minerals administered in an amount within the RDA and/or present in the powder in an amount within the RDA.

A particularly preferred embodiment of the powder comprises a buffer salt comprising at least one phosphate salt, the powder further comprising at least one glycine or functional derivative thereof, at least one N-acetylcysteine or functional derivative thereof, Vitamin E, Vitamin C, Vitamin B2 and Zinc and optionally Selenium, most preferably with the vitamins and minerals administered in an amount within the RDA and/or present in the powder in an amount within the RDA.

As a non-limiting example, a unit dosage form of the powder can comprise about 600 mg N-acetyl cysteine (NAC), about 600 mg Glycine, one or more phosphate salts (e.g., about 500 mg total phosphate salt), and the RDA of each of Vitamin E, Vitamin C, Vitamin B2 and Zinc.

As another non-limiting example, a unit dosage form of the powder can comprise about 485 mg N-acetyl cysteine (NAC), about 485 mg Glycine, one or more phosphate salts (e.g., about 500 mg total phosphate salt), and the RDA of each of Vitamin E, Vitamin C, Vitamin B2, Selenium and Zinc

Subject to the requirement that the powder comprises a phosphate salt, non-limiting examples of suitable buffer salts which the powder can comprise include citrates; phosphates such as monophosphates, diphosphates, triphosphates, and polyphosphates (e.g., magnesium phosphate and trisodium phosphate); hexametaphosphate; carbonates such as monocarbonates and bicarbonates (e.g., magnesium carbonate, sodium monocarbonate, sodium bicarbonate, potassium bicarbonate and potassium monocarbonate); and mixtures thereof. Preferably the buffer salt comprises at least one of dibasic sodium phosphate, monobasic potassium phosphate, dipotassium phosphate, monobasic calcium phosphate, dicalcium phosphate, or tricalcium phosphate; most preferably at least one of disodium phosphate or potassium phosphate dibasic.

The powder can be formed by drying a liquid. The drying can comprise spray-drying, freeze-drying or any other procedure of drying known in the art. The present disclosure is not limited to a specific embodiment of the drying by which the powder is made, and the drying can be any process that decreases moisture, e.g., to form a solid composition. Additionally or alternatively, the powder can be made by dry mixing.

The powder has at least one characteristic selected from the group consisting of (i) the amino acids are present in an amount that at least partially comprises the amino acid in a form selected from the group consisting of free form, dipeptides, tripeptides and mixtures thereof and (ii) the powder is substantially free of enzymes and antibodies. For example, at least a portion of the amino acids can be free form, i.e., not covalently bound to any other compound. Additionally or alternatively, at least a portion of the amino acids can be present in one or more of di-peptides; tri-peptides; larger peptides (i.e., at least four amino acid residues therein) or intact proteins. Nevertheless, in a preferred embodiment, the powder is substantially free of therapeutic proteins such as enzymes and/or antibodies, and in a particular embodiment, completely free of therapeutic proteins such as enzymes and/or antibodies. The powder is substantially free (or completely free) of proteins of four or more amino acid residues. "Substantially free" means less than 1.0 wt.%, preferably less than 0.5 wt.%, more preferably less than 0.2 wt.%, even more preferably less than 0.1 wt.%, most preferably less than 0.01 wt.%. In some embodiments, the powder consists essentially of (or consists of) any residual water (e.g., after drying), the buffer salt, the amino acids, the vitamins and mineral.

The powder can be reconstituted in a liquid such as water to form a nutritional product such as a beverage, preferably at room temperature and/or without applying heat. In a preferred embodiment, the powder can be reconstituted in a liquid comprising a component selected from the group consisting of an acidic component (e.g., a juice and/or a polyphenol such as a flavonoid) and a protein (e.g., milk protein such as casein and/or whey; plant protein; collagen; and blends thereof). For example, the powder can be reconstituted in an oral nutrition supplement (ONS) such as the commercially available products MERITENE^{®}, BOOST^{®}, NUTREN^{®} and SUSTAGEN^{®}. Nevertheless, the present disclosure is not limited to a specific embodiment of the liquid in which the powder is reconstituted, and the liquid can be any liquid suitable for consumption by an animal or human.

Further in this regard, the present disclosure is not limited to a specific embodiment of the acidic component in the liquid, and the acidic component can be any compound that provides acidity to the liquid. Still further, the present disclosure is not limited to a specific embodiment of the protein in the liquid, and the protein can be any protein, particularly those types that are subject to denaturation or gelation upon a pH increase.

The powder can be provided to the consumer in a container (e.g., a sealed container) for reconstitution in the container and/or for allowing the user to pour the powder from the container into a drinking receptacle in which the powder is reconstituted. Non-limiting examples of suitable containers include bags, boxes, cartons, bottles, or combinations thereof. Preferred containers include a sachet/stick pack, i.e., a small disposable pouch, typically of flexible film such as cellophane or paper, preferably capable of being torn open at one or both ends, and containing one unit dosage form of the powder (i.e., one serving of the powder).

The combination of at least one glycine or functional derivative thereof and at least one N-acetylcysteine or functional derivative thereof can be provided by any of the compositions disclosed by U.S. Patent Nos. 8,362,080, 8,802,730 and 9,084,760, each entitled "Increasing glutathione levels for therapy," and U.S. Patent App. Pub. No. 2018/0161297 entitled "Benefits of Supplementation with N-Acetylcysteine and Glycine to Improve Glutathione Levels".

Methods disclosed herein (not part of the invention) can include the reconstitution step. The reconstitution step is preferably performed by an individual who is the recipient of the administering and/or performs the administering, and/or another individual acting under the direction of individual who is the recipient of the administering and/or performs the administering. The liquid in which the powder is reconstituted preferably contains an acidic component (e.g., juice and/or a polyphenol such as a flavonoid) and/or a protein (e.g., a milk protein such as whey and/or casein; plant protein; collagen; and blends thereof).

A kit (not part of the invention) can be provided comprising at least one glycine or functional derivative thereof, at least one N-acetylcysteine, L-cysteine or functional derivative thereof, and buffer salt in one or more container. In some embodiments, one or more of these compounds can be isolated compounds. The kit preferably includes instructions for reconstituting the mixture in a liquid comprising at least one of an acidic component (e.g., juice and/or a polyphenol such as a flavonoid) or a protein (e.g., a milk protein such as whey and/or casein; plant protein; collagen; and blends thereof).

In such a kit, the at least one glycine or functional derivative thereof, the at least one N-acetylcysteine, L-cysteine or functional derivative thereof, and the buffer salt can be provided together in one or more prepackaged unit dosage forms, for example in separate containers that each contain a dried powder such that each container contains one prepackaged unit dosage form.

Such a kit can comprise a plurality of compositions for admixing together to form one or more of the powders disclosed herein. For example, the kit can contain two or more dried powders in separate containers relative to each other, the separate powders each containing a portion of the final unit dosage form.

As a non-limiting example of such a kit, the kit can contain one or more first containers that house the at least one glycine or functional derivative thereof and can also contain one or more second containers that house the at least one N-acetylcysteine or functional derivative thereof. The content of one of the first containers can be admixed with one of the second containers to form at least a portion of the unit dosage form of the composition. In such a case, the kit can have a configuration selected from the group consisting of (i) the buffer salt is provided by the selected first container, (ii) the buffer salt is provided by the selected second container, (iii) a portion of the buffer salt is provided by the selected first container and another portion of the buffer salt is provided by the selected second container, and (iv) the buffer salt is provided by at least one of a plurality of third containers.

As used herein, a "functional derivative" of glycine is a glycine derivative that is effective in an individual in conjunction with N-acetylcysteine or a functional derivative thereof to increase intracellular GSH levels. A "functional derivative" of N-acetylcysteine is an N-acetylcysteine derivative that is effective in an individual in by itself or in conjunction with glycine (or a functional derivative thereof) to increase intracellular GSH levels.

The glycine is preferably L-glycine and/or L-glycine ethyl ester. Non-limiting examples of suitable glycine functional derivatives include D-Allylglycine; N-[Bis(methylthio)methylene]glycine methyl ester; Boc-allyl-Gly-OH (dicyclohexylammonium) salt; Boc-D-Chg-OH; Boc-Chg-OH; (R)-N-Boc-(2'-chlorophenyl)glycine; Boc-L-cyclopropylglycine; Boc-L-cyclopropylglycine; (R)-N-Boc-4-fluorophenylglycine; Boc-D-propargylglycine; Boc-(S)-3-thienylglycine; Boc-(R)-3-thienylglycine; D-a-Cyclohexylglycine; L-a-Cyclopropylglycine; N-(2-fluorophenyl)-N-(methylsulfonyl)glycine; N-(4-fluorophenyl)-N-(methylsulfonyl)glycine; Fmoc-N-(2,4-dimethoxybenzyl)-Gly-OH; N-(2-Furoyl)glycine; L-a-Neopentylglycine; D-Propargylglycine; sarcosine; Z-a-Phosphonoglycine trimethyl ester, and a mixture thereof.

The powder and/or the resultant nutritional product (not part of the invention) can be administered at least one day per week, preferably at least two days per week, more preferably at least three or four days per week (e.g., every other day), most preferably at least five days per week, six days per week, or seven days per week. The time period of administration can be at least one week, preferably at least one month, more preferably at least two months, most preferably at least three months, for example at least four months. In an embodiment, dosing is at least daily; for example, a subject may receive one or more doses daily. In some embodiments, the administration continues for the remaining life of the individual.

The glycine and the N-acetylcysteine or L-cysteine or functionel derivative may be formulated in a particular ratio. In some embodiments, the formulation may comprise these components in the following exemplary ratios: 1:1, 1:2, 1:3, 1:4, 1:5, 1:6, 1:7, 1:8, 1:9, 1:10, 1:12, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, 1:150, 1:200, 1:300, 1:400, 1:500, 1:600, 1:750, 1:1000, and 1:10,000, and each of these ratios can be GLY:NAC in some embodiments and NAC:GLY in other embodiments. In particular embodiments, the formulation may comprise these components in the following weight percentages (either the same for both glycine and the N-acetylcysteine or different weight percentages for each): 1%, 2%, 3%, 4%, 5%, 6%, 7%, 8%, 9%, 10%, 12%, 15%, 20%, 25%, 30%, 40%, 45%, 50%, 55%, 60%, 65%, 70%, 75%, 80%, 85%, 90%, 95%, 97%, or 99%, for example.

The N-acetylcysteine or functional derivative thereof can be administered in an amount of about 0.1 - 100 milligram (mg) of N-acetylcysteine (NAC) or functional derivative thereof per kilogram (kg) of body weight of the subject. The glycine (GLY) or functional derivative thereof can be administered in an amount of about 0.1 - 100 milligram (mg) of glycine or functional derivative thereof per kilogram (kg) of body weight of the subject. In some embodiments, these amounts are provided at least partially by a dipeptide comprising both the N-acetylcysteine or functional derivative thereof and the glycine or functional derivative thereof.

In a particular non-limiting example, the daily doses for a 60 kg subject can be as follows:
NAC or functional derivative thereof: 6 to 6,000 mg/day
GLY or functional derivative thereof: 6 to 6,000 mg/day

The ratio of the buffering salts to the amino acids can depend on the particular application of the resultant product (e.g., whether the reconstituted liquid is a beverage or some other composition category), the serving size of the resultant product, and the protein content of the resultant product. However, one of skill in this art will be able to use the disclosures herein to determine the appropriate ratio of the buffering salts to the amino acids by considering these factors.

The powder and/or the resultant nutritional product (not part of the invention) disclosed herein is preferably administered to the subject orally. As such, non-limiting examples of the form of the composition include natural foods, processed foods, natural juices, concentrates and extracts, liposomes, and sustained-release preparations.

The powder and/or the resultant nutritional product (not part of the invention) disclosed herein can use any of a variety of formulations for therapeutic administration. More particularly, pharmaceutical compositions can comprise appropriate pharmaceutically acceptable carriers or diluents and may be formulated into preparations in solid, semi-solid, liquid or gaseous forms. As such, administration of the composition can be achieved in various ways, but preferably oral.

In pharmaceutical dosage forms, the compounds may be administered as their pharmaceutically acceptable salts. They may also be used in appropriate association with other pharmaceutically active compounds. The following methods and excipients are merely exemplary and are in no way limiting.

For oral preparations, the compounds can be used alone or in combination with appropriate additives to make tablets, powders, or granules, for example, with conventional additives, such as lactose, mannitol, maltodextrin, corn starch or potato starch; with binders, such as crystalline cellulose, cellulose functional derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

### EXAMPLE (powders not according to the invention)

The following non-limiting example conducted by the present inventors further support the compounds, compositions, and methods disclosed herein.

### Background

Standard protein or amino acids in powder format do not have buffer salts, except for "therapeutic/active" proteins such as enzymes or antibodies. For example, these particular compounds are not stable in pure form and thus need additional salts to stabilize. In contrast, amino acids are provided without buffer salts.

Buffer salts are also common in many ready-to-drink protein applications with the additional use of basic solutions, such as potassium hydroxide or sodium hydroxide, because ready-to-drink beverages are typically heat-treated. Nevertheless, powder products that will be reconstituted in room temperature or cold temperature liquids immediately before consumption are not provided with buffer salts.

Therefore, the present inventors investigated a solution for a reconstitution of amino acids such as glycine and/or NAC in a liquid that is acidic and/or contains protein, e.g., for at-home reconstitution of an amino acid powder.

### Experiments

The present inventors performed reconstitution of various amino acid powders at room temperature, without heat processing. The amino acids were blended with or without buffer salts and then poured into the drink.

Specifically, 600 mg N-acetyl cysteine (NAC) and 600 mg Glycine were added into 100 ml water and mixed. Then 27.5 g of an unflavoured powder containing milk protein was added and mixed. In another sample, 200 mg disodium phosphate and 300 mg potassium phosphate dibasic were blended with 600 mg NAC and 600mg Glycine. The blend was then added into 100 ml water and mixed. After this, 27.5 gram of the unflavoured powder containing milk protein was added and mixed. Use of disodium phosphate and potassium phosphate dibasic significantly decreased protein denaturation and precipitation.

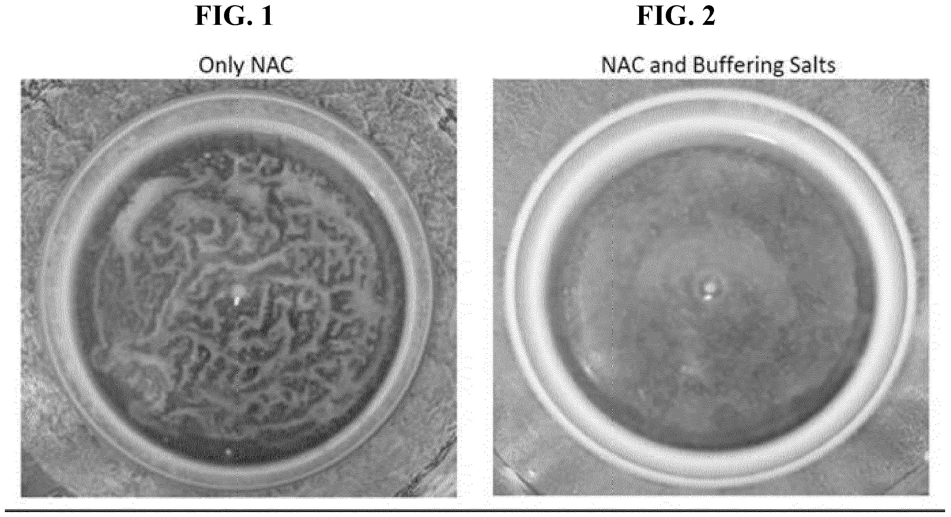

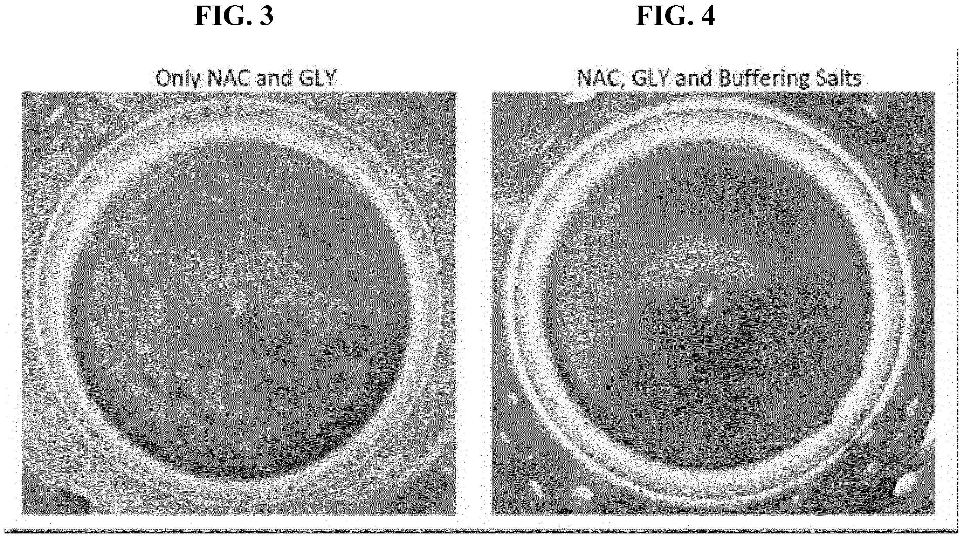

In this regard, the photographs **FIGS. 1-4** show the bottom of the cup. **FIG. 1** shows the result of reconstitution of powdered NAC without buffer salts, **FIG. 2** shows the result of reconstitution of powdered NAC with buffer salts, **FIG. 3** shows the result of reconstitution of powdered NAC and glycine without buffer salts, and **FIG. 4** shows the result of reconstitution of powdered NAC and glycine with buffer salts. As shown in the figures, residues are present when the buffer salts are absent, and the presence of buffer salts avoids precipitation.

## Claims

1. A powder comprising a phosphate salt, at least one glycine or functional derivative thereof, at least one N-acetylcysteine, L-cysteine or functional derivative thereof, Vitamin E, Vitamin C, Vitamin B2 and Zinc, the powder comprising less than 1.0 wt.% of proteins of four or more amino acid residues, and the powder having at least one characteristic selected from the group consisting of (i) the amino acids in the powder are present in an amount that at least partially comprises the amino acid in a form selected from the group consisting of free form, dipeptides, tripeptides and mixtures thereof and (ii) the powder is substantially free of enzymes and antibodies.

2. The powder according to Claim 1, wherein the powder is in a unit dosage form comprising each of the Vitamin E, the Vitamin C, the Vitamin B2 and the Zinc in an amount within the recommended daily dietary allowance.

## Patentansprüche

1. Pulver, umfassend ein Phosphatsalz, mindestens ein Glycin oder ein funktionelles Derivat davon, mindestens ein N-Acetylcystein, L-Cystein oder ein funktionelles Derivat davon, Vitamin E, Vitamin C, Vitamin B2 und Zink, das Pulver umfassend zu weniger als 1,0 Gew.-% Proteine von vier oder mehr Aminosäureresten und wobei das Pulver mindestens eine Eigenschaft aufweist, die aus der Gruppe ausgewählt ist, bestehend aus (i) die Aminosäuren in dem Pulver sind in einer Menge vorhanden, die die Aminosäure mindestens teilweise in einer Form umfasst, die aus der Gruppe ausgewählt ist, bestehend aus einer Freiform, Dipeptiden, Tripeptiden und Mischungen davon, und (ii) das Pulver ist im Wesentlichen frei von Enzymen und Antikörpern.

2. Pulver nach Anspruch 1, wobei das Pulver in einer Einheitsdosierungsform vorliegt, umfassend jedes von dem Vitamin E, dem Vitamin C, dem Vitamin B2 und dem Zink in einer Menge, die innerhalb der empfohlenen täglichen Nährstoffzufuhr liegt.

## Revendications

1. Poudre comprenant un sel de phosphate, au moins une glycine ou un dérivé fonctionnel de celle-ci, au moins une N-acétylcystéine, une L-cystéine ou un dérivé fonctionnel de celle-ci, de la vitamine E, de la vitamine C, de la vitamine B2 et du zinc, la poudre comprenant moins de 1,0 % en poids de protéines de quatre résidus ou plus d'acides aminés, et la poudre ayant au moins une caractéristique choisie dans le groupe constitué (i) des acides aminés dans la poudre qui sont présents en une quantité qui comprend au moins partiellement l'acide aminé sous une forme choisie dans le groupe constitué de forme libre, de dipeptides, de tripeptides et de mélanges de ceux-ci et (ii) de la poudre qui est substantiellement exempte d'enzymes et d'anticorps.

2. Poudre selon la revendication 1, dans laquelle la poudre est présentée sous forme de dose unitaire comprenant chacune de la vitamine **E,** de la vitamine C, de la vitamine B2 et du zinc en une quantité comprise dans l'apport nutritionnel journalier recommandé.
